# EUROPEAN PATENT APPLICATION

(11) **EP 4 559 720 A1**
(43) Date of publication of application: **28.05.2025**
(21) Application number: 24201667.3
(22) Date of filing: 20.09.2024
(51) Int. Cl.: B60L 3/00, B60L 50/64, H01M 10/42, H01M 10/48, H01M 10/60, H01M 10/613, H01M 10/625

(54) **ABNORMALITY DETERMINATION DEVICE AND ABNORMALITY DETERMINATION METHOD**

(30) Priority: 19.10.2023 JP 2023180397
(71) Applicant: TOYOTA JIDOSHA KABUSHIKI KAISHA, Aichi-ken, 471-8571 (JP)
(72) Inventor: MIGITA, Tsubasa, Toyota-shi, Aichi-ken, 471-8571 (JP); OGUMA, Yasumasa, Toyota-shi, Aichi-ken, 471-8571 (JP); MORIKAWA, Kei, Toyota-shi, Aichi-ken, 471-8571 (JP); UKITA, Keisuke, Toyota-shi, Aichi-ken, 471-8571 (JP); KURUMA, Yusuke, Toyota-shi, Aichi-ken, 471-8571 (JP)
(74) Representative: Cabinet Beau de Loménie

(57) **Abstract**

An ECU executes a process including: acquiring a result of detection (S100); determining whether a vehicle is being stopped (S102); determining, when the vehicle is being stopped (YES in S102), whether an output value of a hydrogen sulfide sensor is a first threshold value or more (S104); turning on a warning light when the output value is the first threshold value or more (YES in S104); determining, when the vehicle is not being stopped (NO in S102), whether the output value of the hydrogen sulfide sensor is a second threshold value or more (S106); and turning on a warning light (S108) when the output value is the second threshold value or more (YES in S106).

## Description

### CROSS REFERENCE TO RELATED APPLICATIONS

This nonprovisional application is based on Japanese Patent Application No. 2023-180397 filed on October 19, 2023 with the Japan Patent Office, the entire contents of which are hereby incorporated by reference and to which the person of the art can refer when considering the present disclosure.

### BACKGROUND

### Field

The present disclosure relates to an abnormality determination device for a battery pack.

### Description of the Background Art

A technique is known according to which a detection device for detecting the concentration of hydrogen sulfide is disposed in a battery pack including a solid-state battery for which a sulfide-based material is used, and the presence or absence of hydrogen sulfide is detected by means of the detection device to determine whether the battery pack has an abnormality.

Regarding such a technique, Japanese Patent Laying-Open No. 2015-041598 for example discloses a technique, according to which concentration information acquisition means for acquiring information on the concentration of hydrogen sulfide is provided in a housing of a battery pack, and presence or absence of leakage of hydrogen sulfide is detected using the acquired information on the concentration.

### SUMMARY

Cooling, temperature increase, or the like of the battery pack causes non-uniform temperature of the air in the housing, to thereby cause convection of the air in the housing. Accordingly, when gas containing hydrogen sulfide is generated in the housing, the gas is likely to be stirred and diluted by convection. Therefore, hydrogen sulfide may not be detected with high accuracy by means of the detection device. As a result, it may not be possible to determine with high accuracy whether the battery pack has an abnormality.

An object of the present disclosure is to provide an abnormality determination device and an abnormality determination method to determine with high accuracy whether a battery pack has an abnormality.

An abnormality determination device according to an aspect of the present disclosure includes: a battery pack mounted on a mobile object, and including a sulfide-based all-solid-state battery and a housing in which the sulfide-based all-solid-state battery is housed; a hydrogen sulfide sensor that outputs a signal indicating a concentration of hydrogen sulfide in the battery pack; and a determination unit that determines whether the battery pack has an abnormality, by using an output value of the hydrogen sulfide sensor. The determination unit determines that the battery pack has an abnormality, when the output value is larger than a threshold value, and determines whether the battery pack has an abnormality, by using, as the threshold value, a value that varies depending on a state of the mobile object.

Thus, since the degree of convection of air in the battery pack varies depending on the state of the mobile object, a value that varies depending on the state of the mobile object can be employed as the threshold value and the output value of the hydrogen sulfide sensor can be used, to determine whether the battery pack has an abnormality, and accordingly, it is possible to determine, with high accuracy, whether the battery pack has an abnormality.

In the present embodiment, a cooler that cools the battery pack is mounted on the mobile object. While the cooler is in operation, the determination unit determines that the battery pack has an abnormality, when the output value is larger than a first threshold value and, while the cooler is not in operation, the determination unit determines that the battery pack has an abnormality, when the output value is larger than a second threshold value. The second threshold value is larger than the first threshold value.

Thus, while the cooler is in operation, convection of air in the battery pack is less likely to occur. Therefore, hydrogen sulfide residing in the battery pack can be detected with high accuracy, and it is possible to determine with high accuracy that the battery pack has an abnormality, when the output value is larger than the first threshold value.

Further, in the present embodiment, the mobile object includes a vehicle. While the vehicle is stopped, the determination unit determines that the battery pack has an abnormality, when the output value is larger than a first threshold value and, while the vehicle travels, the determination unit determines that the battery pack has an abnormality, when the output value is larger than a second threshold value. The second threshold value is larger than the first threshold value.

Thus, since the battery pack generates less heat while the vehicle is stopped, convection of air in the battery pack is less likely to occur. Therefore, hydrogen sulfide residing in the battery pack can be detected with high accuracy, and it is possible to determine with high accuracy that the battery pack has an abnormality, when the output value is larger than the first threshold value.

Further, in the present embodiment, when a vehicle stoppage time of the vehicle having elapsed from transition of the vehicle from a system-stopped state to a system-activated state is a predetermined time or less and the output value is larger than the first threshold value, the determination unit determines that the battery pack has an abnormality.

Thus, while the vehicle is in the state where convection of air in the battery pack does not occur, hydrogen sulfide can be detected with high accuracy, and therefore, it is possible to determine with high accuracy that the battery pack has an abnormality, when the output value is larger than the first threshold value.

Further, in the present embodiment, the abnormality determination device further includes a notification unit that makes a notification, when the determination unit determines that the battery pack has an abnormality, of occurrence of the abnormality.

Thus, the notification unit can be used to make a notification of the abnormality, and therefore, it is possible to notify the user of the abnormality of the battery pack.

Further, in the present embodiment, the mobile object includes a vehicle. The determination unit is activated each time a predetermined time has elapsed while the vehicle is in a system-stopped state, and determines that the battery pack has an abnormality, when the output value is larger than the threshold value.

Thus, while the vehicle is in the state where convection of air in the battery pack does not occur, hydrogen sulfide can be detected with high accuracy and it can be determined with high accuracy whether the battery pack has an abnormality.

Further, in the present embodiment, the abnormality determination device further includes a communication unit capable of communicating with a terminal. When the determination unit determines that the battery pack has an abnormality, the communication unit transmits, to the terminal, information that the battery pack has the abnormality.

Thus, the communication unit can be used to transmit, to the terminal, information that the battery pack has an abnormality, and thereby inform a user of the vehicle of the abnormality of the battery pack.

Further, in the present embodiment, the battery pack is capable of undergoing external charging by an external power supply external to the vehicle. The determination unit determines whether the battery pack has an abnormality, while the external charging of the battery pack is performed.

Thus, while the vehicle is in the state where convection of air in the battery pack does not occur, hydrogen sulfide can be detected with high accuracy and it can be determined with high accuracy whether the battery pack has an abnormality.

Further, in the present embodiment, the abnormality determination device further include: a communication unit capable of communicating with a terminal; and a notification unit. When the determination unit determines that the battery pack has an abnormality, the determination unit makes a notification that the battery pack has the abnormality, by using the notification unit, and transmits, to the terminal, information that the battery pack has the abnormality, by using the communication unit.

Thus, it is possible to notify the vehicle's user of the abnormality of the battery pack, by making the notification that the battery pack has the abnormality by means of the notification unit, and transmitting to the terminal the information that the battery pack has the abnormality by means of the communication unit.

An abnormality determination method according to another aspect of the present disclosure is an abnormality determination method for a battery pack mounted on a mobile object, and including a sulfide-based all-solid-state battery and a housing in which the sulfide-based all-solid-state battery is housed. The abnormality determination method includes: outputting a signal indicating a concentration of hydrogen sulfide in the battery pack; determining whether the battery pack has an abnormality, by using an output value of the concentration; determining that the battery pack has an abnormality, when the output value is larger than a threshold value; and determining whether the battery pack has an abnormality, by using, as the threshold value, a value that varies depending on a state of the mobile object.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

Fig. 1 is a diagram schematically showing an overall configuration of a vehicle on which a battery pack is mounted.
Fig. 2 is a diagram showing a schematic configuration of the battery pack.
Figs. 3A and 3B are diagrams illustrating a schematic configuration of a unit cell according to the present embodiment.
Fig. 4 is a diagram showing functional blocks of an ECU.
Fig. 5 is a flowchart illustrating an example of a process executed by the ECU according to a modification.
Fig. 6 is a flowchart (1) illustrating an example of a process executed by the ECU according to a modification.
Fig. 7 is a flowchart (2) illustrating an example of a process executed by the ECU according to a modification.
Fig. 8 is a flowchart (3) illustrating an example of a process executed by the ECU according to a modification.
Fig. 9 is a flowchart (4) illustrating an example of a process executed by the ECU according to a modification.
Fig. 10 is a flowchart (5) illustrating an example of a process executed by the ECU according to a modification.

### DESCRIPTION OF THE PREFERRED EMBODIMENTS

Embodiments of the present disclosure are described hereinafter in detail, with reference to the drawings. In the drawings, the same or corresponding parts are denoted by the same reference characters, and a description thereof is not herein repeated.

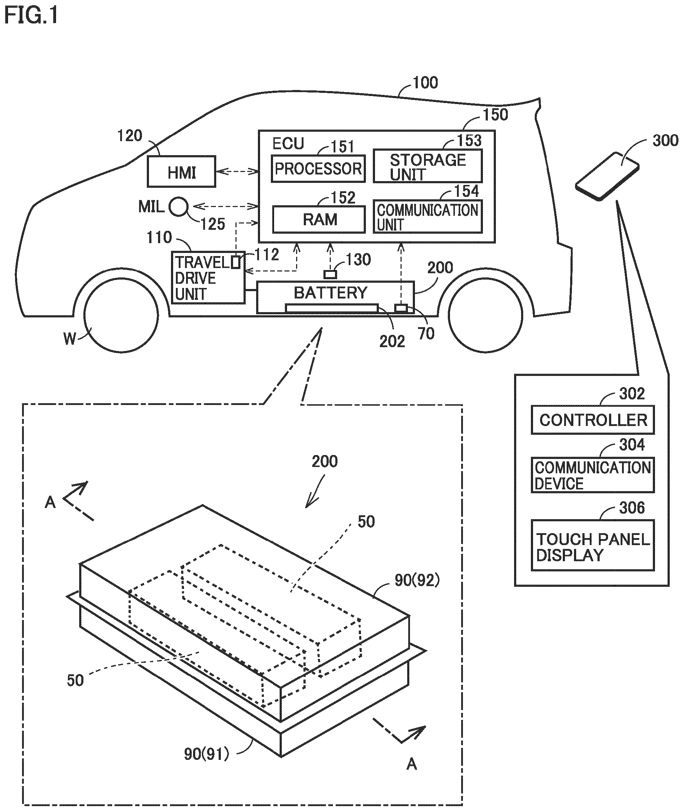

Fig. 1 is a diagram schematically showing an overall configuration of a vehicle 100 on which a battery pack 200 is mounted. Vehicle 100 includes battery pack 200 that stores electric power for traveling. Vehicle 100 is configured to be capable of traveling with electric power stored in battery pack 200. In connection with the present embodiment, while a case is described, by way of example, where vehicle 100 is a battery electric vehicle (BEV) without an engine (internal combustion engine), vehicle 100 may be a hybrid electric vehicle (HEV) or a plug-in hybrid electric vehicle (PHEV) with an engine. Battery pack 200 is provided with a cooler 202. Cooler 202 includes, for example, a cooling passage configured to be able to exchange heat with a battery module in battery pack 200, and a pump (not shown) that circulates a cooling liquid in the cooling passage.

Vehicle 100 includes a controller (ECU: Electronic Control Unit) 150. ECU 150 is configured to be capable of performing charging control and discharging control for battery pack 200. ECU 150 includes a processor 151, a RAM (Random Access Memory) 152, a storage unit 153, and a communication unit 154. RAM 152 functions as a working memory that temporarily stores data to be processed by processor 151. Storage unit 153 stores a program as well as information (for example, maps, mathematical expressions, and various parameters) to be used for the program. When processor 151 executes the program stored in storage unit 153, various types of control (charging control and discharging control for battery pack 200, cooling control by means of cooler 202, and communication control by means of communication unit 154) in ECU 150 are executed. Communication unit 154 is configured to be capable of performing wireless communication with an external terminal 300 in a predetermined manner of communication.

When cooler 202 is operated by, for example, operating a pump to circulate a cooling liquid in the cooling passage, ECU 150 sets a flag to the ON state, indicating that cooler 202 is in operation and, when cooler 202 is stopped, ECU 150 sets the flag to the OFF state.

Terminal 300 includes a controller 302, a communication device 304, and a touch panel display 306. Controller 302 includes a processor and a storage device such as memory. Communication device 304 is configured to be capable of wireless communication with communication unit 154 of vehicle 100. Touch panel display 306 includes a display unit and an input unit (both are not shown). A program in the memory is executed by the processor to cause controller 302 of terminal 300 to perform various control processes for terminal 300.

A monitoring module 130 includes various sensors that detect a state (for example, voltage, current, and temperature) of battery pack 200 (a battery module 50), and outputs the results of the detection to ECU 150. Battery pack 200 is charged with regenerative electric power from a travel drive unit 110 described later herein, or charged with electric power supplied from a charging facility external to vehicle 100 (external charging).

Vehicle 100 further includes travel drive unit 110, an HMI (Human Machine Interface) device 120, an MIL (Malfunction Indicator Lamp) 125, and a driving wheel W. Travel drive unit 110 includes a PCU (Power Control Unit) and an MG (Motor Generator) (not shown), and is configured to drive the MG using electric power stored in battery pack 200 to cause vehicle 100 to travel. The MG is configured to perform regenerative power generation and supply the generated power to battery pack 200. Travel drive unit 110 includes a vehicle speed sensor 112. Vehicle speed sensor 112 detects the speed Va of vehicle 100 (hereinafter referred to as vehicle speed), and transmits the result of the detection to ECU 150. Instead of vehicle speed sensor 112, a wheel speed sensor that detects the rotational speed (wheel speed) of driving wheel W may detect and transmit the wheel speed to ECU 150, and ECU 150 may use the wheel speed to calculate vehicle speed Va, or a rotation speed sensor that detects the rotation speed of the MG may detect and transmit the rotation speed to ECU 150, and ECU 150 may use the rotation speed to calculate vehicle speed Va. Alternatively, a current sensor that detects electric current flowing in the MG may detect and transmit the electric current to ECU 150, and ECU 150 may use the electric current to calculate vehicle speed Va.

HMI device 120 includes an input device and a display device. HMI device 120 may include a touch panel display. MIL 125 includes various warning lights disposed on an instrument panel. MIL 125 includes, for example, a warning light indicating that battery pack 200 has an abnormality.

Battery pack 200 includes a battery case 90 and battery module 50 contained in battery case 90. Battery case 90 includes a lower case 91 and an upper case 92. In the present embodiment, two battery modules 50 are contained in the space formed by lower case 91 and upper case 92. Battery pack 200 is mounted on the floor of vehicle 100. Battery pack 200 may be mounted inside the compartment of vehicle 100, or may be mounted outside the compartment of vehicle 100.

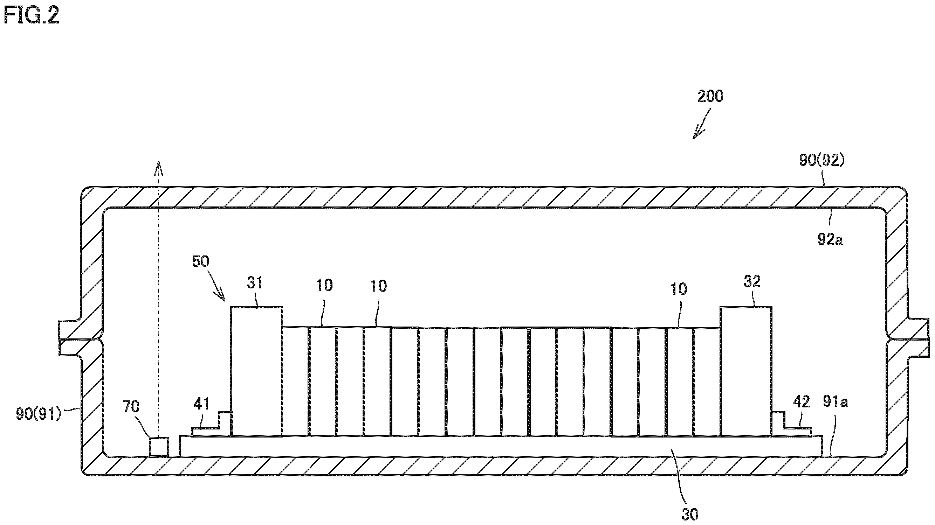

Fig. 2 is a diagram showing a schematic configuration of battery pack 200. Fig. 2 shows a cross section along A-A in Fig. 1. Battery module 50 is a battery assembly in which a plurality of unit cells 10 are connected together. The plurality of unit cells 10 are stacked between end plates 31 and 32 that constitute a pair.

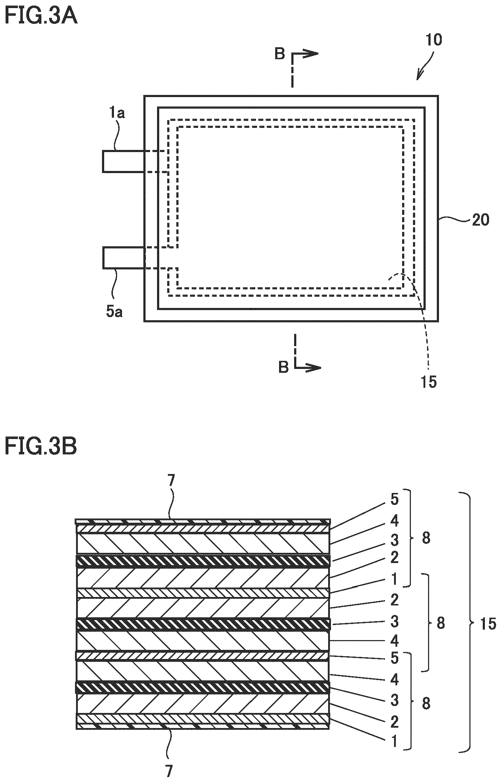

Figs. 3A and 3B are diagrams illustrating a schematic configuration of unit cell 10 according to the present embodiment. Fig. 3A is a top view of unit cell 10. Unit cell 10 is a laminate-type all-solid-state cell in which a laminate film is used as an exterior member 20, and a negative electrode terminal (negative electrode tab) 1a and a positive electrode terminal (positive electrode tab) 5a protrude from exterior member 20. The laminate film may for example be a pouch formed of an aluminum laminate film, and may be a film having a three-layer structure in which an aluminum foil is sandwiched between resin films.

Fig. 3B shows an all-solid-state cell stack 15 contained in exterior member 20, and shows a cross section along B-B in Fig. 3A. All-solid-state cell stack 15 includes three all-solid-state cell elements 8, each made up of a negative electrode current collector layer 1, a negative electrode active material layer 2, a solid electrolyte layer 3, a positive electrode active material layer 4, and a positive electrode current collector layer 5 that are stacked in this order, and the three all-solid-state cell elements 8 are stacked in such a manner that the order in which the layers are stacked is opposite with respect to each other, so as to share negative electrode current collector layer 1 and positive electrode current collector layer 5. Negative electrode current collector layer 1 is connected to negative electrode terminal 1a, and positive electrode current collector layer 5 is connected to positive electrode terminal 5a. The number of all-solid-state cell elements 8 included in all-solid-state cell stack 15 may be one, or may be four or more. An insulating film 7 electrically insulates all-solid-state cell stack 15 from exterior member (laminate film) 20.

Unit cell 10 is a sulfide-based all-solid-state cell. In the present disclosure, the sulfide-based all-solid-state cell refers to a cell in which at least one of the material for positive electrode active material layer 4 and the material for solid electrolyte layer 3 contains a sulfur component. In the present embodiment, solid electrolyte layer 3 contains a sulfide-based solid electrolyte, and the sulfide-based solid electrolyte may be generated for example from diphosphorus pentasulfide (P₂S₅) or lithium sulfide (Li₂S) as a starting material. In this case, positive electrode active material layer 4 may contain, for example, lithium cobaltate, lithium nickelate, lithium iron phosphate, or the like. When solid electrolyte layer 3 is composed of an oxide-based solid electrolyte, a sulfur-based positive electrode active material is used for positive electrode active material layer 4. The sulfur-based positive electrode active material may be an organic sulfur compound or an inorganic sulfur compound. Both solid electrolyte layer 3 and positive electrode active material layer 4 may contain a sulfur component.

Air may enter unit cell 10 from, for example, a sealed portion of exterior member 20 (laminate film). If moisture is contained in this air, the sulfur component contained in solid electrolyte layer 3 or positive electrode active material layer 4 may react with the moisture to generate hydrogen sulfide, which may be released into battery case 90.

Referring to Fig. 2, a plurality of unit cells 10 are arranged and stacked between end plates 31 and 32 that constitute a pair. A plurality of unit cells 10 in the state of being stacked are placed between end plates 31 and 32 of the pair, and a predetermined restraint load is applied to the unit cells by a restraint band or the like (not shown). End plates 31 and 32 of the pair are fixed to a bottom plate 30 by brackets 41 and 42. Battery module 50 including unit cells 10 stacked between end plates 31 and 32 of the pair, and bottom plate 30 is fixed to a bottom surface 91a of lower case 91. Battery case 90 is a housing that houses battery module 50.

A hydrogen sulfide sensor 70 is disposed inside battery case 90. Hydrogen sulfide sensor 70 is a sensor that detects concentration C of hydrogen sulfide (H₂S) contained in the atmosphere (the air in battery pack 200), and outputs a voltage signal indicating the result of the detection to ECU 150. An output value of hydrogen sulfide sensor 70 is hereinafter referred to as "output value Vb." Hydrogen sulfide sensor 70 may for example be a hot wire type semiconductor sensor or a constant potential electrolytic sensor. In the present embodiment, hydrogen sulfide sensor 70 is provided on bottom surface 91a of lower case 91 or in the vicinity of bottom surface 91a.

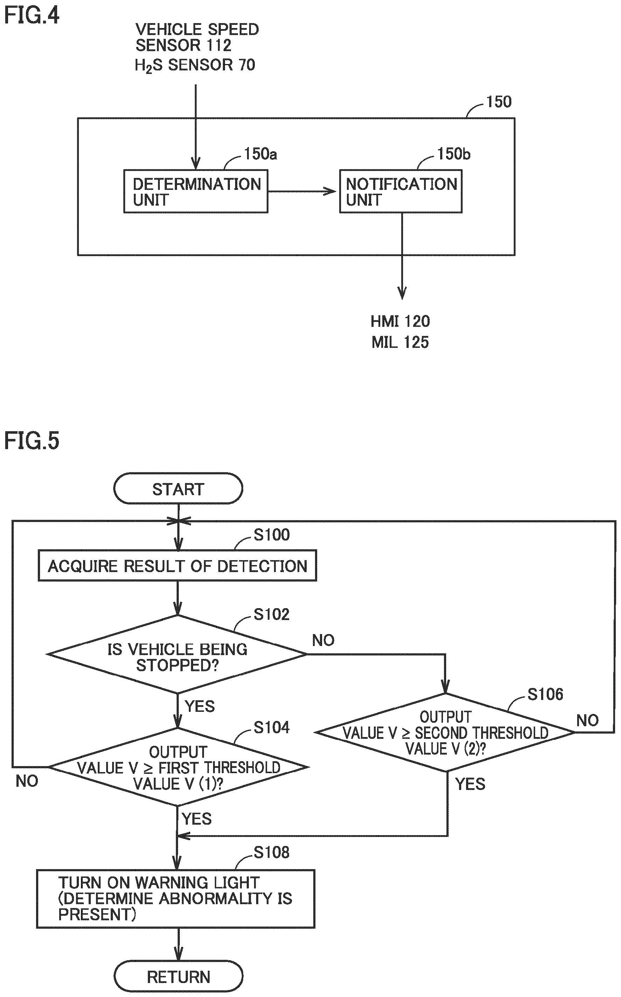

Fig. 4 is a diagram showing functional blocks of ECU 150. ECU 150 includes a determination unit 150a and a notification unit 150b. Determination unit 150a determines whether a predetermined condition is satisfied, based on a voltage signal output from hydrogen sulfide sensor 70 and a voltage signal output from vehicle speed sensor 112. When determination unit 150a determines that the predetermined condition is satisfied, notification unit 150b makes a predetermined notification, by using at least one of HMI device 120 and MIL 125.

Battery pack 200 having the above-described configuration includes the solid-state cells for which a sulfide-based material is used, and therefore, it is possible to determine whether battery pack 200 has an abnormality, by detecting whether hydrogen sulfide is present, by means of hydrogen sulfide sensor 70 disposed in battery case 90. However, the temperature of the air in battery case 90 may become non-uniform, due to cooling, temperature increase, or the like of battery pack 200. Accordingly, convection of the air may occur in battery case 90. As a result, when a gas containing hydrogen sulfide is generated in battery case 90, the gas is likely to be stirred and diluted by convection. Therefore, hydrogen sulfide may not be detected with high accuracy by means of hydrogen sulfide sensor 70. As a result, it may not be possible to determine with high accuracy whether battery pack 200 has an abnormality.

In view of this, in the present embodiment, determination unit 150a determines that battery pack 200 has an abnormality, when output value Vb of hydrogen sulfide sensor 70 is larger than a threshold value, and determines whether battery pack 200 has an abnormality by using, as the threshold value, a value that varies depending on the state of the mobile object. More specifically, determination unit 150a determines that battery pack 200 has an abnormality, when output value Vb of hydrogen sulfide sensor 70 is larger than a first threshold value Vb (1) and, while vehicle 100 travels, determination unit 150a determines that battery pack 200 has an abnormality, when output value Vb is larger than a second threshold value Vb (2). Second threshold value Vb (2) is larger than first threshold value Vb (1).

In this case, the degree of convection of the air in battery pack 200 varies depending on whether vehicle 100 is traveling or being stopped. In particular, while the vehicle is stopped, battery pack 200 generates less heat and therefore, convection of the air is less likely to occur in battery pack 200. Thus, while the vehicle is stopped, the threshold value can be set to the smaller value Vb (1) than that while the vehicle is traveling, to detect hydrogen sulfide in battery pack 200 with high accuracy. As a result, it can be determined with high accuracy whether battery pack 200 has an abnormality.

An example of a process executed by ECU 150 is described hereinafter with reference to Fig. 5. Fig. 5 is a flowchart illustrating an example of a process executed by ECU 150. A series of operations shown in this flowchart is repeatedly executed at predetermined intervals.

In step (step is hereinafter abbreviated as S) 100, ECU 150 acquires results of detection from various sensors. Specifically, ECU 150 acquires the result of detection (output value Vb) by hydrogen sulfide sensor 70 and the result of detection (vehicle speed Va) by vehicle speed sensor 112. The process thereafter proceeds to S102.

In S102, ECU 150 determines whether vehicle 100 is being stopped. For example, when vehicle speed Va detected by vehicle speed sensor 112 falls within a range of speed corresponding to a stopped state equal to or less than threshold value Va (0), ECU 150 determines that vehicle 100 is being stopped. When it is determined that vehicle 100 is being stopped (YES in S102), the process proceeds to S104.

In S104, ECU 150 determines whether output value Vb of hydrogen sulfide sensor 70 is first threshold value Vb (1) or more. First threshold value Vb (1) is an output value of the sensor when concentration C of hydrogen sulfide is a predetermined first value. When it is determined that output value Vb of hydrogen sulfide sensor 70 is first threshold value Vb (1) or more (YES in S104), the process proceeds to S108. When it is determined that the speed of vehicle 100 does not fall within the range of speed corresponding to the stopped state (i.e., falls within a range of speed corresponding to the traveling state) and thus the vehicle is not being stopped (NO in S102), the process proceeds to S106.

In S106, ECU 150 determines whether output value Vb of hydrogen sulfide sensor 70 is second threshold value Vb (2) or more. Second threshold value Vb (2) is an output value of the sensor when concentration C of hydrogen sulfide is a predetermined second value (> first value). When it is determined that output value Vb of hydrogen sulfide sensor 70 is second threshold value Vb (2) or more (YES in S106), the process proceeds to S108. When it is determined that output value Vb of hydrogen sulfide sensor 70 is smaller than first threshold value Vb (1) (NO in S104) or when it is determined that output value Vb of hydrogen sulfide sensor 70 is smaller than second threshold value Vb (2) (NO in S106), the process returns to S100.

In S108, ECU 150 turns on a warning light of MIL 125 to indicate that hydrogen sulfide is contained in the air in battery pack 200. The process thereafter ends.

An example of operation of ECU 150 based on the above-described structure and flowchart is described.

It is supposed, for example, that gas is generated inside any of unit cells 10 included in two battery modules 50 in battery pack 200, due to deterioration or the like, and the generated gas leaks in battery pack 200. As the generated gas leaking out of unit cell 10 increases, the sulfur component contained in the gas also increases. As a result, hydrogen sulfide is generated from the sulfur component of the gas leaking in battery pack 200 and moisture contained in the air in battery pack 200. As the leaking gas increases, concentration C of hydrogen sulfide in battery pack 200 also increases.

When the result of detection by hydrogen sulfide sensor 70 and the result of detection by vehicle speed sensor 112 are acquired (S100), it is determined whether vehicle 100 is being stopped (S102).

### <While vehicle 100 is stopped>

When vehicle speed Va is equal to or less than threshold value Va (0), which corresponds to the vehicle-stopped state, it is determined that vehicle 100 is being stopped (YES in S102), and it is determined whether output value Vb of hydrogen sulfide sensor 70 is first threshold value Vb (1) or more.

While vehicle 100 is stopped, electric power of battery pack 200 is not used, and therefore, there is no factor that makes the temperature of the air in battery pack 200 non-uniform, and convection of the air is less likely to occur. Hydrogen sulfide, which is heavier than air, resides in a lower space in battery pack 200. Therefore, first threshold value Vb (1) can be set smaller than second threshold value Vb (2) to detect hydrogen sulfide with high accuracy. When output value Vb of hydrogen sulfide sensor 70 is first threshold value Vb (1) or more (concentration C is the first value or more) (YES in S104), it is determined that battery pack 200 has an abnormality and a warning light is turned on (S108). When output value Vb of hydrogen sulfide sensor 70 is smaller than first threshold value Vb (1) (NO in S104), the warning light is kept off.

### <While vehicle 100 travels>

When vehicle speed Va is greater than threshold value Va (0), which corresponds to the vehicle travel state, it is determined that vehicle 100 is traveling (NO in S102), and it is determined whether output value Vb of hydrogen sulfide sensor 70 is second threshold value Vb (2) or more.

When battery pack 200 is mounted outside the vehicle compartment, the battery pack may be influenced by a flow of air generated while vehicle 100 is traveling. When battery pack 200 is mounted inside the vehicle compartment, the battery pack may be influenced by air in the vehicle compartment resultant from air conditioning of vehicle 100 while vehicle 100 travels. Because of these influences, convection is likely to be caused by a partial increase or decrease in the temperature of the air in battery pack 200. As a result, the gas containing hydrogen sulfide in battery pack 200 is stirred and diluted. Therefore, second threshold value Vb (2) can be set larger than first threshold value Vb (1) to prevent erroneous detection. When output value Vb of hydrogen sulfide sensor 70 is second threshold value Vb (2) or more (concentration C is the second value or more) (YES in S106), it is determined that battery pack 200 has an abnormality and a warning light is turned on (S 108). When output value Vb of hydrogen sulfide sensor 70 is smaller than second threshold value Vb (2) (NO in S106), the warning light is kept off.

Thus, regarding ECU 150 serving as the abnormality determination device according to the present embodiment, the degree of convection of air in battery pack 200 varies depending on whether vehicle 100 is traveling or being stopped. In particular, while the vehicle is stopped, battery pack 200 generates less heat and therefore, convection of the air is less likely to occur in battery pack 200. Therefore, when gas containing hydrogen sulfide is discharged in battery pack 200, the hydrogen sulfide heavier than air resides in a lower portion of battery pack 200. Accordingly, while the vehicle is stopped, the threshold value can be set smaller than that while the vehicle travels, to thereby detect hydrogen sulfide in battery pack 200 with high accuracy. Thus, it can be determined with high accuracy whether battery pack 200 has an abnormality. In this way, an abnormality determination device and an abnormality determination method can be provided to determine with high accuracy whether a battery pack has an abnormality.

In the following, modifications are described.

According to the above embodiment, vehicle 100 is described as an example of the mobile object, however, the mobile object is not particularly limited to vehicle 100 but may be any mobile object on which battery pack 200 formed of a sulfide-based solid-state battery is mounted. For example, the mobile object may be a railroad vehicle, a ship, an airplane, or the like, instead of vehicle 100.

Further, according to the above embodiment, a case where hydrogen sulfide sensor 70 is placed on the bottom surface of battery case 90 is described by way of example, however, the position where the sensor is placed is not limited to the bottom surface, but may be an upper portion or the side surface of battery case 90.

Further, according to the above embodiment, a case where a single hydrogen sulfide sensor 70 is placed in battery case 90 is described by way of example, however, a plurality of hydrogen sulfide sensors 70 may be placed in battery case 90. For example, the sensors may be placed respectively on two locations that are a front side in battery case 90 (front side in vehicle 100) and a rear side in battery case 90 (rear side in vehicle 100).

Thus, even while vehicle 100 travels on an uphill road or a downhill road, or stops on a sloped road, for example, one of the sensors that is located at a position where hydrogen sulfide resides (the sensor that is located at a lower position in the vertical direction, for example) can be used to detect hydrogen sulfide, so that whether hydrogen sulfide is present can be detected with high accuracy and accordingly it can be determined with high accuracy whether battery pack 200 has an abnormality.

Further, according to the above embodiment, hydrogen sulfide sensor 70 outputs a voltage signal proportional to the concentration, however, hydrogen sulfide sensor 70 may output a first voltage when the concentration is the second value or more, may output a second voltage when the concentration is the first value or more and less than the second value, and may output no signal when the concentration is less than the second value, for example.

Further, according to the above embodiment, it is determined, while vehicle 100 stops, whether output value Vb of hydrogen sulfide sensor 70 is first threshold value Vb (1) or more, and it is determined, while vehicle 100 travels, whether output value Vb of hydrogen sulfide sensor 70 is second threshold value Vb (2) or more, however, the state of vehicle 100 (mobile object) is not limited to the state of stopping and the state of traveling. For example, ECU 150 may determine, while cooler 202 of vehicle 100 is in operation, whether output value Vb of hydrogen sulfide sensor 70 is first threshold value Vb (1) or more, and may determine, while cooler 202 is not in operation, whether output value Vb of hydrogen sulfide sensor 70 is second threshold value Vb (2) or more. In this case, in S102 of Fig. 5, it is determined "whether cooler 202 is in operation," instead of "whether vehicle 100 is being stopped." Since the other steps are similar to those illustrated in Fig. 5, the detailed description thereof is not herein repeated.

In this case as well, hydrogen sulfide residing in battery pack 200 while cooler 202 is in operation can be detected with high accuracy, and therefore, it can be determined with high accuracy that battery pack 200 has an abnormality, when output value Vb is larger than first threshold value Vb (1).

Further, according to the above embodiment, it is determined, while vehicle 100 is stopped, whether output value Vb of hydrogen sulfide sensor 70 is first threshold value Vb (1) or more, however, operation of ECU 150 is not limited to such operation.

For example, ECU 150 may determine whether output value Vb (1) is first threshold value Vb (1) or more, when the following condition is satisfied: a vehicle stoppage time having elapsed from transition of vehicle 100 from a system-stopped state (IG-OFF state) to a system-activated state (IG-ON state) is a predetermined time or less, instead of the condition that vehicle 100 is being stopped. ECU 150 may further determine, when the above condition is not satisfied, whether output value Vb is second threshold value Vb (2) or more. The predetermined time is not particularly limited, as long as it is before energization starts.

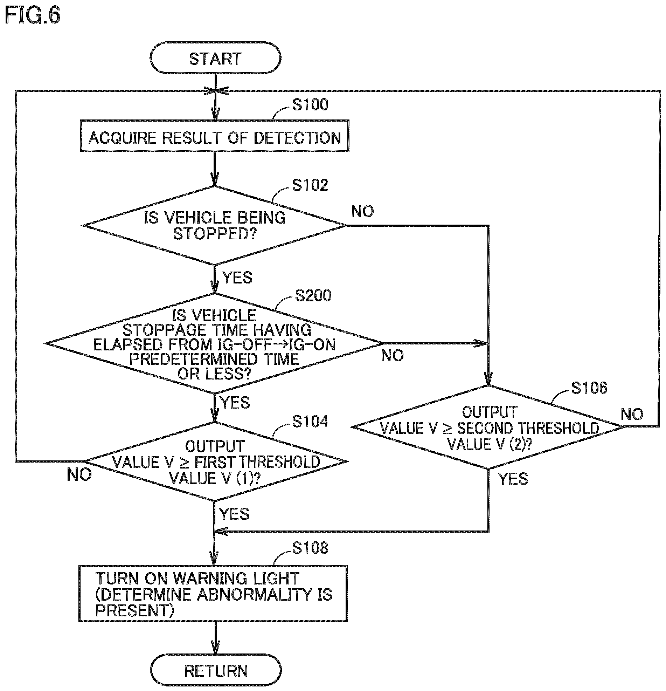

Fig. 6 is a flowchart (1) illustrating an example of a process executed by ECU 150 according to a modification. The process in S100, S102, S104, S106, and S108 in Fig. 6 is the same as the process in S100, S102, S104, S106, and S108 in Fig. 5, except for the following. Therefore, the detailed description thereof is not herein repeated.

When it is determined that vehicle 100 is being stopped (YES in S102), the process proceeds to S200.

In S200, ECU 150 determines whether the vehicle stoppage time after transition from the IG-OFF state to the IG-ON state is a predetermined time or less. ECU 150 uses a timer or the like to measure the elapsed time (vehicle stoppage time) when transition from the IG-OFF to the IG-ON state occurs. When it is determined that the vehicle stoppage time is the predetermined time or less (YES in S200), the process proceeds to S104. When the vehicle stoppage time is more than the predetermined time (NO in S200), the process proceeds to S106.

Thus, it is determined whether output value Vb of hydrogen sulfide sensor 70 is first threshold value Vb (1) or more, particularly when the vehicle stoppage time is the predetermined time or less, and therefore, hydrogen sulfide can be detected with high accuracy before vehicle 100 starts traveling, and thus it can be determined with high accuracy whether battery pack 200 has an abnormality.

Further, according to the above embodiment, it is determined, while vehicle 100 is stopped, whether output value Vb of hydrogen sulfide sensor 70 is first threshold value Vb (1) or more, however, ECU 150 may operate in the following manner. Specifically, ECU 150 determines, while vehicle 100 is stopped and battery pack 200 is externally charged by AC charging, whether output value Vb of hydrogen sulfide sensor 70 is first threshold value Vb (1) or more. ECU 150 determines, while vehicle 100 is stopped and battery pack 200 is externally charged by DC charging, whether output value Vb of hydrogen sulfide sensor 70 is second threshold value Vb (2) or more.

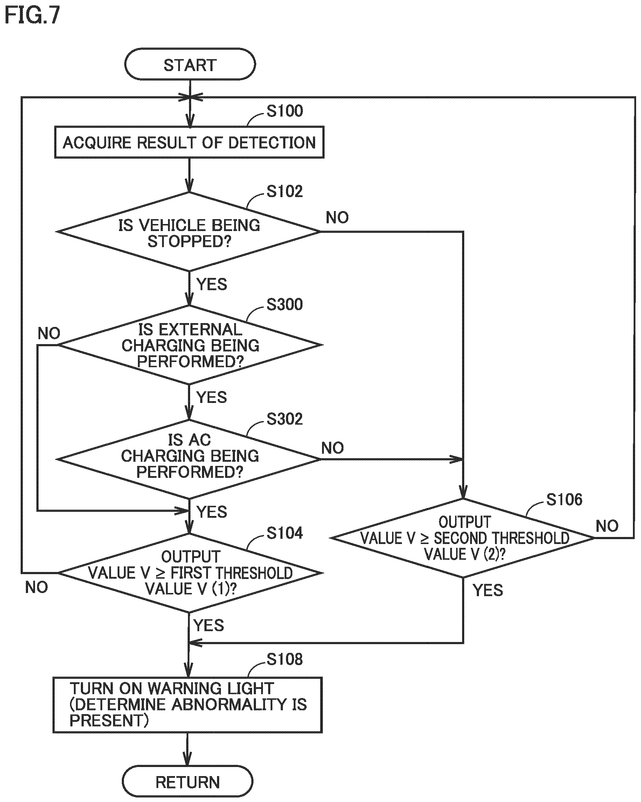

Fig. 7 is a flowchart (2) illustrating an example of a process executed by ECU 150 according to a modification. The process in S100, S102, S104, S106, and S108 in Fig. 7 is the same as the process in S100, S102, S104, S106, and S108 in Fig. 5, except for the following. Therefore, the detailed description thereof is not herein repeated.

When it is determined that vehicle 100 is being stopped (YES in S102), the process proceeds to S300.

In S300, ECU 150 determines whether external charging is being performed. For example, when a connector is attached to an inlet of vehicle 100 (not shown) and charging current flows through battery pack 200, ECU 150 determines that external charging is being performed. For example, the inlet is provided with a switch or a circuit that outputs an ON signal to ECU 150 when the connector is attached to the inlet. ECU 150 determines that the connector is attached to the inlet, when receiving the ON signal from the switch or the circuit. When it is determined that external charging is being performed (YES in S300), the process proceeds to S302.

In S302, ECU 150 determines whether AC charging is being performed. For example, the inlet is provided with a switch or a circuit that outputs to ECU 150 a signal indicating the type of an attached connector, when the connector is attached. For example, ECU 150 determines that AC charging is being performed, when receiving, from the switch or the circuit, a signal indicating that an AC charging connector is attached. For example, ECU 150 determines that AC charging is not being performed (DC charging is being performed), when receiving, from the switch or the circuit, a signal indicating that a DC charging connector is attached. When it is determined that AC charging is being performed (YES in S302), the process proceeds to S104. When it is determined that AC charging is not being performed (NO in S302), the process proceeds to S106. When it is determined that external charging is not being performed (NO in S300), the process proceeds to S104.

During AC charging, the charging time is long and battery pack 200 generates heat slowly, and therefore, the amount of generated heat is smaller than that during DC charging, and convection of air is less likely to occur in battery pack 200. Therefore, hydrogen sulfide can be detected with high accuracy to determine with high accuracy whether battery pack 200 has an abnormality. During DC charging, the amount of heat generated in battery pack 200 is larger than that during AC charging, and convection of air may occur in battery pack 200. Therefore, erroneous detection can be prevented by determining whether output value Vb of hydrogen sulfide sensor 70 is second threshold value Vb (2) or more.

Further, according to the above embodiment, a warning light is turned on when battery pack 200 has an abnormality, however, at least a notification of the abnormality of battery pack 200 may be given to a user, and the manner of giving the notification is not limited to turn-on of the warning light. For example, a warning in the form of character information or an image may be indicated on a display device of a touch panel display of HIM 120, or the user may be notified of the abnormality of battery pack 200, through a warning sound or a sound for warning that battery pack 200 has an abnormality, from a speaker or the like (not shown).

Further, according to the above embodiment, when battery pack 200 has an abnormality, the user is notified of the abnormality of battery pack 200 through turn-on of a warning light, however, the manner of notifying the user is not limited to use of a device in vehicle 100, but, for example, information indicating that battery pack 200 has an abnormality may be transmitted to terminal 300 of the user, in addition to turning on the warning light. Specifically, when it is determined that output value Vb of hydrogen sulfide sensor 70 is the threshold value (Vb (1) or Vb (2)) or more, ECU 150 turns on the warning light and transmits information indicating that battery pack 200 has an abnormality to terminal 300 through communication unit 154. Upon receiving the information indicating that battery pack 200 has an abnormality, terminal 300 displays the information on touch panel display 306 of terminal 300.

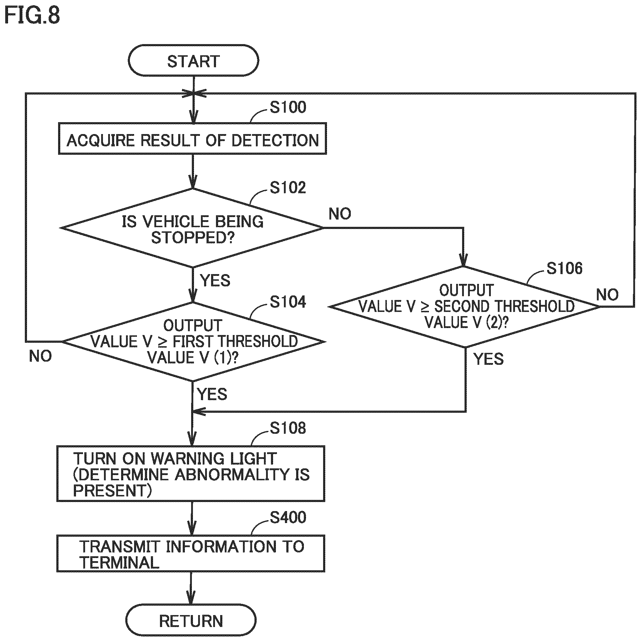

Fig. 8 is a flowchart (3) illustrating an example of a process executed by ECU 150 according to a modification. The process in S100, S102, S104, S106, and S108 in Fig. 8 is the same as the process in S100, S102, S104, S106, and S108 in Fig. 5, except for the following. Therefore, the detailed description thereof is not herein repeated.

When it is determined that output value Vb of hydrogen sulfide sensor 70 is first threshold value Vb (1) or more (YES in S104), or when it is determined that output value Vb is second threshold value Vb (2) or more (YES in S106), the process proceeds to S400 after a warning light is turned on (S108).

In S400, ECU 150 transmits information indicating that battery pack 200 has an abnormality to a terminal (e.g., terminal 300) registered in advance, by using communication unit 154. The process thereafter ends.

Thus, it is possible to notify a user that battery pack 200 has an abnormality, when the user is located far from vehicle 100, for example, during external charging.

Further, according to the above embodiment, it is determined, while vehicle 100 is stopped, whether the output value of hydrogen sulfide sensor 70 is the first threshold value or more, however, while the system of vehicle 100 is stopped, the system of vehicle 100 may be activated each time a predetermined time has elapsed and it may be determined whether output value Vb of hydrogen sulfide sensor 70 is first threshold value Vb (1) or more.

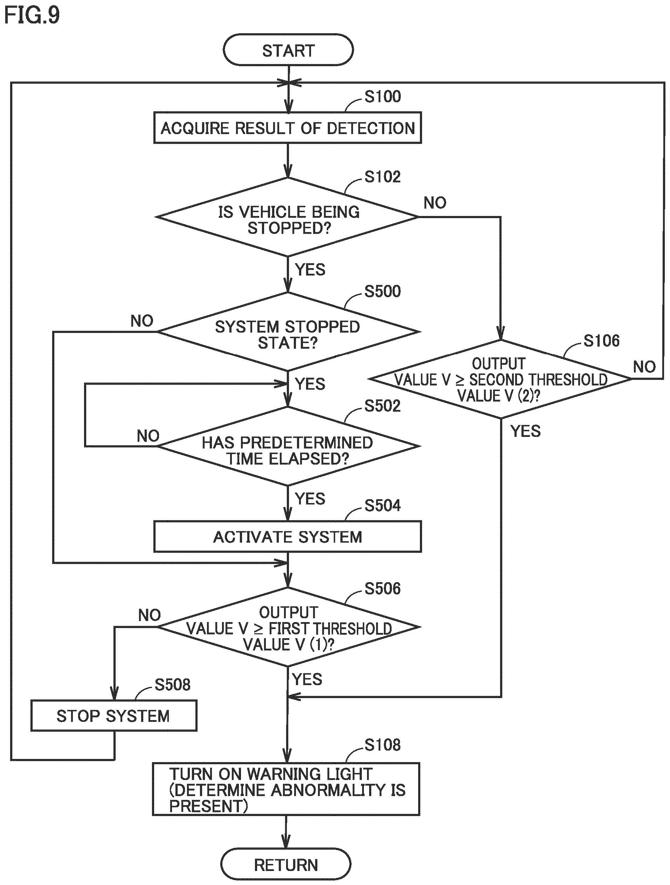

Fig. 9 is a flowchart (4) illustrating an example of a process executed by ECU 150 according to a modification. The process in S100, S102, S106, and S108 in Fig. 9 is the same as the process in S100, S102, S106, and S108 in Fig. 5, except for the following. Therefore, the detailed description thereof is not herein repeated.

When it is determined that vehicle 100 is being stopped (YES in S102), the process proceeds to S500.

In S500, ECU 150 determines whether the system of vehicle 100 is in the stopped state (IG-OFF state). When it is determined that the system of vehicle 100 is in the stopped state (YES in S500), the process proceeds to S502.

In S502, ECU 150 determines whether the duration of the stopped state is a predetermined time or more. When it is determined that the duration of the stopped state is the predetermined time or more (YES in S502), the process proceeds to S504.

In S504, ECU 150 changes the system from the stopped state to the activated state (IG-ON state). The process thereafter proceeds to S506.

In S506, ECU 150 determines whether output value Vb of hydrogen sulfide sensor 70 is first threshold value Vb (1) or more. When it is determined that output value Vb is first threshold value Vb (1) or more (YES in S506), the process proceeds to S108. When it is determined that output value Vb is less than first threshold value Vb (1) (NO in S506), the process proceeds to S508.

In S508, ECU 150 changes the system of vehicle 100 from the activated state to the stopped state. The process thereafter returns to S100.

Thus, when the system of vehicle 100 is in the stopped state, the system is changed to the activated state each time a predetermined time has elapsed, and whether hydrogen sulfide has been generated, i.e., whether battery pack 200 has an abnormality, can be detected.

Further, according to the above embodiment, it is determined, while vehicle 100 travels, whether output value Vb is second threshold value Vb (2) or more, regardless of cooling of battery pack 200, however, it may be determined, while vehicle 100 travels and cooler 202 is in operation, whether output value Vb is first threshold value Vb (1) or more and, it may be determined, while vehicle 100 travels and cooler 202 is not in operation, whether output value Vb is second threshold value Vb (2) or more.

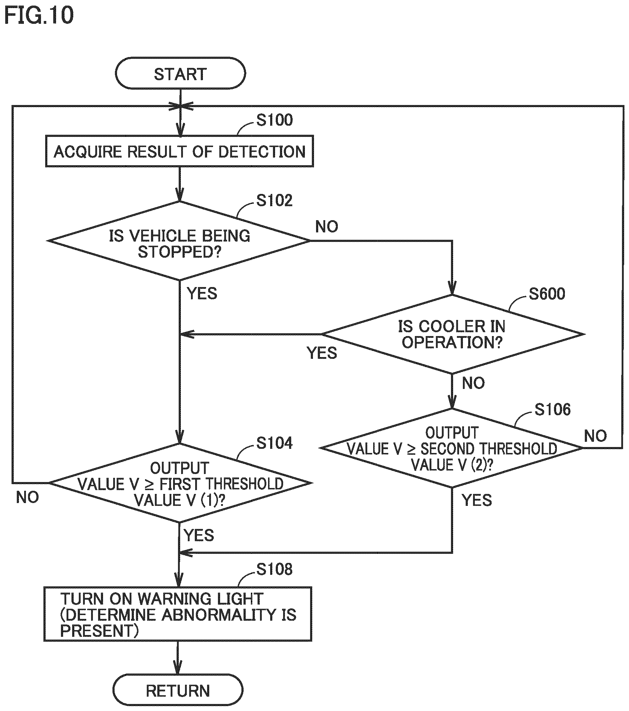

Fig. 10 is a flowchart (5) illustrating an example of a process executed by ECU 150 according to a modification. The process in S 100, S102, S104, S106, and S108 in Fig. 10 is the same as the process in S100, S102, S104, S106, and S108 in Fig. 5, except for the following. Therefore, the detailed description thereof is not herein repeated.

When it is determined that vehicle 100 is not being stopped (NO in S102), the process proceeds to S600.

In S600, ECU 150 determines whether cooler 202 is in operation. For example, when a flag for indicating that cooler 202 is in operation is in the ON state, ECU 150 determines that cooler 202 is in operation. When it is determined that cooler 202 is in operation (YES in S600), the process proceeds to S104. When it is determined that cooler 202 is not in operation (NO in S600), the process proceeds to S106.

Thus, even when vehicle 100 is traveling, there is no nonuniformity of the temperature and convection does not occur as long as cooler 202 is in operation, so that it can be detected with high accuracy whether hydrogen sulfide has been generated.

The above described modifications may be implemented in the form of being entirely or partially combined.

Although the present disclosure has been described and illustrated in detail, it is clearly understood that the same is by way of illustration and example only and is not to be taken by way of limitation, the scope of the present disclosure being interpreted by the terms of the appended claims.

## Claims

1. An abnormality determination device comprising:
a battery pack (200) to be mounted on a mobile object (100), and including a sulfide-based all-solid-state battery (50) and a housing (90) in which the sulfide-based all-solid-state battery is housed;
a hydrogen sulfide sensor (70) configured to output a signal indicating a concentration of hydrogen sulfide in the battery pack (200); and
a determination unit (150a) configured to determine whether the battery pack (200) has an abnormality, by using an output value of the hydrogen sulfide sensor (70), wherein
the determination unit (150a) is configured to
determine that the battery pack (200) has an abnormality, when the output value is larger than a threshold value, and
determine whether the battery pack (200) has an abnormality, by using, as the threshold value, a value that varies depending on a state of the mobile object (100).

2. The abnormality determination device according to claim 1, wherein
a cooler (202) configured to cool the battery pack (200) is mounted on the mobile object (100),
while the cooler (202) is in operation, the determination unit (150a) is configured to determine that the battery pack (200) has an abnormality, when the output value is larger than a first threshold value,
while the cooler (202) is not in operation, the determination unit (150a) is configured to determine that the battery pack (200) has an abnormality, when the output value is larger than a second threshold value, and
the second threshold value is larger than the first threshold value.

3. The abnormality determination device according to claim 1, wherein
the mobile object (100) comprises a vehicle (100),
while the vehicle (100) is stopped, the determination unit (150a) is configured to determine that the battery pack (200) has an abnormality, when the output value is larger than a first threshold value,
while the vehicle (100) travels, the determination unit (150a) is configured to determine that the battery pack (200) has an abnormality, when the output value is larger than a second threshold value, and
the second threshold value is larger than the first threshold value.

4. The abnormality determination device according to claim 3, wherein when a vehicle stoppage time of the vehicle (100) having elapsed from transition of the vehicle (100) from a system-stopped state to a system-activated state is a predetermined time or less and the output value is larger than the first threshold value, the determination unit (150a) is configured to determine that the battery pack (200) has an abnormality.

5. The abnormality determination device according to claim 4, wherein the abnormality determination device (150) further comprises a notification unit (150b) configured to make a notification, when the determination unit (150a) determines that the battery pack (200) has an abnormality, of occurrence of the abnormality.

6. The abnormality determination device according to claim 1, wherein
the mobile object (100) comprises a vehicle (100), and
the determination unit (150a) is configured to be activated each time a predetermined time has elapsed while the vehicle (100) is in a system-stopped state, and to determine that the battery pack (200) has an abnormality when the output value is larger than the threshold value.

7. The abnormality determination device according to claim 6, wherein
the abnormality determination device (150) further comprises a communication unit (154) capable of communicating with a terminal (300), and
when the determination unit (150a) determines that the battery pack (200) has an abnormality, the communication unit (154) is configured to transmit, to the terminal (300), information that the battery pack (200) has the abnormality.

8. The abnormality determination device according to claim 3, wherein
the battery pack (200) is capable of undergoing external charging by an external power supply external to the vehicle (100), and
the determination unit (150a) is configured to determine whether the battery pack (200) has an abnormality, while the external charging of the battery pack (200) is performed.

9. The abnormality determination device according to claim 8, wherein
the abnormality determination device (150) further comprises:
a communication unit (154) capable of communicating with a terminal (300); and
a notification unit (150b), and
when the determination unit (150a) determines that the battery pack (200) has an abnormality, the determination unit is configured to make a notification that the battery pack (200) has the abnormality, by using the notification unit (150b), and to transmit, to the terminal (300), information that the battery pack (200) has the abnormality, by using the communication unit (154).

10. An abnormality determination method for a battery pack (200) mounted on a mobile object (100), and including a sulfide-based all-solid-state battery (50) and a housing (90) in which the sulfide-based all-solid-state battery is housed, the abnormality determination method comprising:
outputting a signal indicating a concentration of hydrogen sulfide in the battery pack (200);
determining whether the battery pack (200) has an abnormality, by using an output value of the concentration;
determining that the battery pack (200) has an abnormality, when the output value is larger than a threshold value; and
determining whether the battery pack (200) has an abnormality, by using, as the threshold value, a value that varies depending on a state of the mobile object (100).
